# EUROPEAN PATENT APPLICATION

(11) **EP 4 285 976 A1**
(43) Date of publication of application: **06.12.2023**
(21) Application number: 22176570.4
(22) Date of filing: 31.05.2022
(51) Int. Cl.: A61M 16/04, A61M 16/22, A61M 16/00

(54) **TRACHEAL TUBE WITH SEALING CUFF COMPARTMENTS**

(60) Divisional application: 23170168.1
(71) Applicant: HyperCuff GmbH, 91054 Erlangen (DE)
(72) Inventor: Göbel, Lothar, 91054 Erlangen (DE)
(74) Representative: Hafner & Kohl PartmbB

(57) **Abstract**

A tracheal tube (1) for ventilation of a patient, said tracheal tube (1) comprising a tube shaft (2) having a central lumen (3) to provide the ventilation of the patient in respiratory cycles of the patient by a ventilator of a breathing apparatus connected to the tube shaft(2);a sealing cuff (6) surrounding the tube shaft (2) and being inflatable with a fluid to seal off the patient's trachea in an inflated state of the sealing cuff (6) at a compression area where the sealing cuff (6) is pressed against the mucosa (10) of the patient's trachea, wherein the sealing cuff (6) is divided into torus-shaped sealing cuff compartments (6A,6B) which are adapted to exchange fluid with each other through a fluid passage (6C) during the ventilation of the patient such that the compression area is moved along the mucosa (10) of the patient's trachea during a respiratory cycle of the patient.

## Description

### FIELD OF THE INVENTION

The invention relates to a tracheal tube with sealing cuff compartments used for ventilation of a patient by a breathing apparatus, and in particular to a tracheal tube having a control- and data interface for providing monitoring signals to a connected breathing apparatus.

### TECHNICAL BACKGROUND

Tracheal tubes are catheters that can be inserted into the trachea of a patient and can be used for mechanical or assisted ventilation of the patient and/or for administering anesthetics or drugs to the patient. Tracheal tubes can be used for airway management in the settings of general anesthesia, critical care, mechanical ventilation or emergency medicine.

Most tracheal tubes comprise an inflatable sealing cuff. A sealing cuff is used to seal the trachea and bronchial tree against air leakage as well as against aspiration of gastric contents, blood, or secretions. Such tracheal tubes provide a leak-proved connection between the patient's lung and a ventilator of the breathing apparatus without causing undue pressure. Tracheal tubes can be attached to the breathing apparatus via tapered connectors. Tracheal tubes are used to control the flow of air or other gases through the patient's trachea. Such tracheal tubes can include endotracheal tubes or tracheostomy tubes. An endotracheal tube is a specific type of tracheal tube that is nearly always inserted through the mouth (orotracheal) or nose (nasotracheal) of the patient. A tracheostomy tube is another type of tracheal tube which can be inserted into a tracheostomy stoma to maintain a patent lumen. To create a seal between the tube and the tracheal wall of the trachea an inflatable sealing cuff is used. When having been inflated, the sealing cuff can prevent air from flowing into or out of the patient's lungs except via the passage of the tube. In this manner, major air leaks during positive pressure ventilation, i.e. when air is being pushed into the lungs of the patients as well as gas leaks during anesthesia procedures, can be prevented. The extent of sealing depends on the internal cuff pressure within the inflated sealing cuff. The inflated sealing cuff applies mechanical pressure to the patient's trachea. However, if the applied mechanical pressure on the trachea is too high it may damage the tracheal tissue and may cause a so-called scar tissue stenosis.

To avoid this, tracheal tubes with elongated sealing cuffs have been developed which, when fully inflated, have a diameter that exceeds the diameter of the trachea substantially. The so-called high-volume/low-pressure cuffs allow to reduce the applied pressure to provide an enlarged contact area between the inflated sealing cuff and the surface of the patient's trachea. For example, US 7849857 B2 describes a sealing cuff having an inflatable balloon with a larger diameter than the diameter of the trachea such that the inflated balloon, when inserted into the trachea, assumes a toroidal shape to provide a tight seal even when the ambient pressure above or below the sealing cuff does exceed the pressure inside the inflated balloon.

However, these conventional high-volume/low-pressure sealing cuffs are prone to the formation of open channels or grooves at the contact area between the sealing cuff and the sidewall of the patient's trachea. This happens when the balloon's outer wall folds or curls since the balloon of the sealing cuff is not fully inflated when placed in the trachea. Proliferation of liquids through these open channels will lead to a ventilator-associated pneumonia (VAP).

As a consequence, a sealing cuff for tracheal tubes has been proposed in EP 3838322 A1 which prevents formation of unwanted channels at the contact interface between the sealing cuff at the surface of the patient's trachea. The sealing cuff comprises an expandable body configured to be expanded from a collapsed state to an expanded state wherein the expandable body comprises a specific shape. The expandable body of this conventional sealing cuff has a center portion which comes into contact with the sidewall of the patient's trachea in its expanded state. On expansion, the expandable body of the sealing cuff comes into contact with the surface of the patient's trachea sidewall and thus does fix the tracheal tube in place, i.e. such that the tracheal tube cannot move within the trachea of the patient.

An disadvantage of such a conventional arrangement is that the sealing cuff exerts in its expanded state a relative high mechanical pressure for a long time period on a specific compression point of the trachea's sidewall during the ongoing ventilation of the patient. The duration of the ventilation of a patient can last from several hours (in case of surgery) to months and even years (in case of intensive medical care) .Consequently, to avoid damage of the tracheal tissue of the patient's trachea, the physical pressure within the inflated sealing cuff has to be permanently beneath a predefined threshold pressure of around 20 to 25 cm H2O. Accordingly, with such a conventional inflatable sealing cuff, the pressure within the inflated sealing cuff has to be closely monitored to avoid a cuff pressure beyond the admissible pressure threshold to avoid damaging the tracheal tissue at the compression point where the inflated sealing cuff does press against the trachea's sidewall of the patient.

Accordingly, it is an object of the present invention to provide a tracheal tube with an inflatable sealing cuff which overcomes the above-mentioned problems and which allows to apply at least temporarily a higher sealing cuff pressure without causing any damage to the tracheal tissue of the patient's trachea during ventilation of the patient.

This object is achieved by a tracheal tube with sealing cuff compartments comprising the features of claim 1.

### SUMMARY

The invention provides according to a first aspect a tracheal tube for ventilation of a patient, said tracheal tube comprising
a tube shaft having a central lumen to provide the ventilation of the patient in respiratory cycles of the patient by a ventilator of a breathing apparatus connected to the tube shaft,
a sealing cuff surrounding the tube shaft and being inflatable with a fluid to seal off the patient's trachea in an inflated state of the sealing cuff at a compression area where the sealing cuff is pressed against the mucosa of the patient's trachea,
wherein the sealing cuff is divided into torus-shaped sealing cuff compartments which are adapted to exchange fluid with each other through a fluid passage during the ventilation of the patient such that the compression area is moved along the mucosa of the patient's trachea during a respiratory cycle of the patient.

Accordingly, with the tracheal tube according to the first aspect of the present invention, the compression area between the inflated sealing cuff and the tracheal tissue of the patient's trachea is moving and is not stationary. This allows to apply a higher cuff pressure within the inflated sealing cuff during ventilation of the patient since the compression area is moving along the inner wall of the patient's trachea. The use of a higher sealing cuff pressure leads to an improved sealing of the patient's trachea during ventilation. Moreover, since the compression area is not fixed but does move along the inner surface of the patient's trachea, a high pressure within the inflated sealing cuff does not damage the tracheal tissue and avoids any kind of stenosis. If the sealing pressure within the inflated sealing cuff does locally and for a short time period exceed the allowable sealing cuff pressure threshold, this does not hurt the patient. Consequently, the tracheal tube according to the first aspect of the present invention is less safety-critical and provides more tolerance against the application of a high sealing cuff pressure during ventilation of the patient.

The movement of the compression point or compression area of the inflated sealing cuff during ventilation of the patient has the further advantage that the mucosal blood flow within the mucosa of the patient's trachea is stimulated.

In a possible embodiment of the tracheal tube, the tracheal tube comprises at least one fluid inflation channel which is used to inflate the sealing cuff compartments of the sealing cuff with the fluid after insertion of the tracheal tube into the patient's trachea before starting ventilation of the patient.

In a preferred embodiment of the tracheal tube according to the present invention, the inflation channel has for each sealing cuff compartment of the sealing cuff an associated internal fluid channel opening.

The fluid channel opening forms a passage between the fluid channel and the respective sealing cuff compartment and is adapted to support the exchange of the fluid between the sealing cuff compartments of the sealing cuff during ventilation of the patient.

Accordingly, the provision of the internal fluid channel opening can support the exchange of fluid between the torus-shaped sealing cuff compartments of the sealing cuff to support a movement of the compression area along the mucosa of the patient's trachea during a respiratory cycle of the ventilated patient.

In a further possible embodiment of the tracheal tube according to the first aspect of the present invention, the tracheal tube comprises at least one constriction element configured to divide the sealing cuff into the torus-shaped sealing cuff compartments.

In a further possible embodiment of the tracheal tube according to the first aspect of the present invention, the compression area is formed over time by alternating sealing cuff compartments of the sealing cuff during a respiratory cycle of the ventilated patient.

In a possible embodiment of the tracheal tube according to the first aspect of the present invention, the fluid used for inflating the sealing cuff compartments comprises a liquid or gas. The gas may also comprise a narcotic gas.

In a further possible embodiment of the tracheal tube according to the first aspect of the present invention, a physical pressure within the sealing cuff compartments of the sealing cuff is controlled by a control unit of a breathing apparatus by controlling an inhalation valve and/or an exhalation valve connected to the sealing cuff compartments of the sealing cuff through the fluid inflation channel of the tracheal tube.

In a still further possible embodiment of the tracheal tube according to the first aspect of the present invention, a physical pressure within the inflated sealing cuff is changed dynamically over time during ventilation of the patient by the ventilator of the breathing apparatus through the tube shaft.

In a further possible embodiment of the tracheal tube according to the first aspect of the present invention, the fluid inflation channel comprises at its proximate end a controllable valve to switch the fluid inflation channel between an inflation actuator adapted to inflate the sealing cuff of the tracheal tube after insertion of the tracheal tube into the patient's trachea through the inflation channel and a pressure sensor of the breathing apparatus adapted to supply a cuff pressure signal representing a pressure change of a physical pressure within the sealing cuff to a control unit of the breathing apparatus during ventilation of the patient by the ventilator of the breathing apparatus through the tube shaft of the tracheal tube.

In a possible embodiment of the tracheal tube according to the first aspect of the present invention, the tracheal tube comprises at least one integrated pressure sensor element configured to generate a cuff pressure signal (CPS) representing a pressure change of a physical pressure within the sealing cuff during ventilation of the patient.

In a still further possible embodiment of the tracheal tube according to the first aspect of the present invention, the tracheal tube comprises a first signal interface adapted to supply the cuff pressure signal generated by the integrated pressure sensor element of the tracheal tube to a control unit of the breathing apparatus being connected to the tracheal tube.

In a still further possible embodiment of the tracheal tube according to the first aspect of the present invention, the constriction element of the tracheal tube is adapted to induce an electrical signal in a wire loop integrated in the tube shaft or attached to the tube shaft in response to a lateral oscillating movement of the constriction element along the tube shaft during ventilation of the patient.

In a still further possible embodiment of the tracheal tube according to the first aspect of the present invention, the tracheal tube comprises a second signal interface connected to the wire loop and adapted to supply the induced electrical signal representing the lateral oscillating movement of the constriction element along the tube shaft as a cuff movement signal (CMS) to a control unit of the breathing apparatus connected to the tracheal tube.

In a still further possible embodiment of the tracheal tube according to the first aspect of the present invention, the constriction element comprises a constriction ring including an electrical conductive material.

In a further possible embodiment of the tracheal tube according to the first aspect of the present invention, the sealing cuff of the tracheal tube comprises towards a pulmonary side a first sealing cuff compartment and towards an oral side a second sealing cuff compartment, wherein during an inhalation phase of the respiratory cycle, the patient's trachea is sealed off by the first sealing cuff compartment of the sealing cuff and during an exhalation phase of the respiratory cycle, the patient's trachea is sealed off by the second sealing cuff compartment of the sealing cuff.

In a further possible embodiment of the tracheal tube according to the first aspect of the present invention, the tracheal tube comprises an endotracheal tube.

In a further possible embodiment of the tracheal tube according to the first aspect of the present invention, the tracheal tube comprises a tracheostomy tube.

In a still further possible embodiment of the tracheal tube according to the first aspect of the present invention, the sealing cuff is replaceable in an uninflated state of the sealing cuff.

In a further possible embodiment of the tracheal tube according to the first aspect of the present invention, a maximum volume of the sealing cuff in its inflated state is adjustable by a clamping mechanism.

In a further possible embodiment of the tracheal tube according to the first aspect of the present invention the tracheal tube comprises a third signal interface connected to a pressure sensor integrated in the central lumen of the tube shaft and adapted to supply a ventilation pressure signal (VPS) to a control unit of the breathing apparatus connected to the tracheal tube.

In a further possible embodiment of the tracheal tube according to the first aspect of the present invention the first signal interface for the cuff pressure signal (CPS) and/or the second signal interface for the cuff movement signal (CMS) and/or the third signal interface for the ventilation pressure signal (VPS) form part of a data- and control interface of the tracheal tube adapted to supply these signals as monitoring signals to a breathing apparatus.

In a further possible embodiment of the tracheal tube according to the first aspect of the present invention the tracheal tube comprises a display unit connectable to the data and control interface of the tracheal tube and adapted to display the cuff pressure signal (CPS), the cuff movement signal (CMS) and/or the ventilation pressure signal (VPS

The invention provides according to a second aspect a breathing apparatus comprising the features of claim 21.

The invention provides according to a second aspect a breathing apparatus comprising a ventilator used for ventilation of a patient through a tracheal tube according to the first aspect of the present invention, wherein said breathing apparatus comprises a control unit configured to control the ventilator, an inhalation valve and an exhalation valve of the breathing apparatus and comprising a display unit adapted to display a cuff pressure signal (CPS) representing a pressure change of a physical pressure within the sealing cuff of the tracheal tube connected to the breathing apparatus during ventilation of the patient and/or adapted to display a cuff movement signal (CMS) representing the lateral movement of the sealing cuff along the trachea during ventilation of the patient and/or adapted to display a ventilation air pressure signal (VPS) representing a pressure of the ventilation air measured in a central lumen of the tracheal tube.

### BRIEF DESCRIPTION OF FIGURES

In the following, possible embodiments of the different aspects of the present invention are described in more detail with reference to the enclosed figures.
- Fig. 1: shows a schematic diagram for illustrating a possible exemplary embodiment of a tracheal tube according to the first aspect of the present invention;
- Fig. 2: shows a cross section view for illustrating an operation of a tracheal tube during an inhalation phase of a respiratory cycle;
- Fig. 3: shows a cross section view to illustrate the operation of a tracheal tube during an exhalation phase of the respiratory cycle;
- Fig. 4: illustrates a further exemplary embodiment of a tracheal tube according to the first aspect of the present invention;
- Fig. 5: illustrates a possible exemplary embodiment of a breathing apparatus according to the second aspect of the present invention;
- Figs. 6A to 6G: illustrate the deformation of a sealing cuff of a tracheal tube according to the first aspect of the present invention during ventilation of a patient and a waveform of a corresponding pressure signal.

### DETAILED DESCRPTION OF EMBODIMENTS

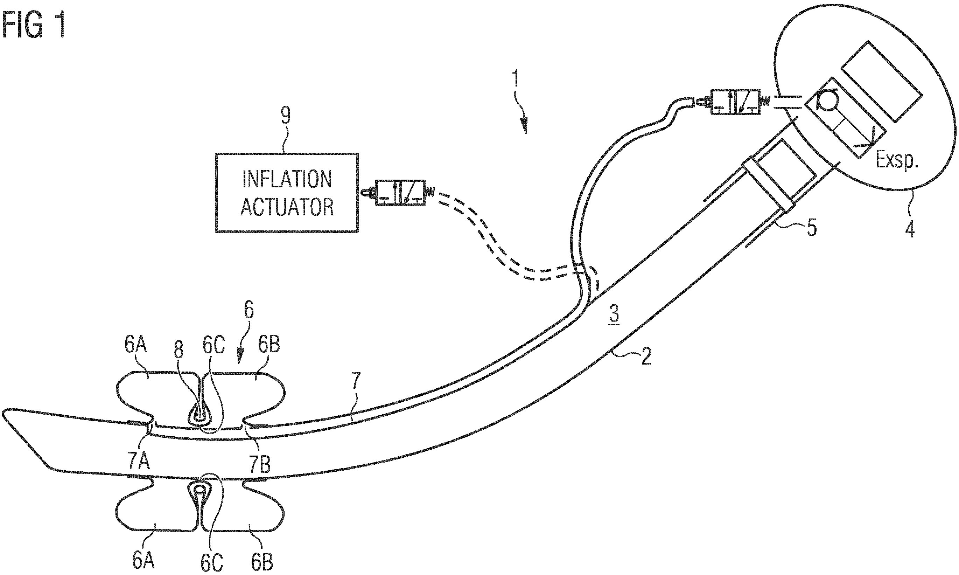

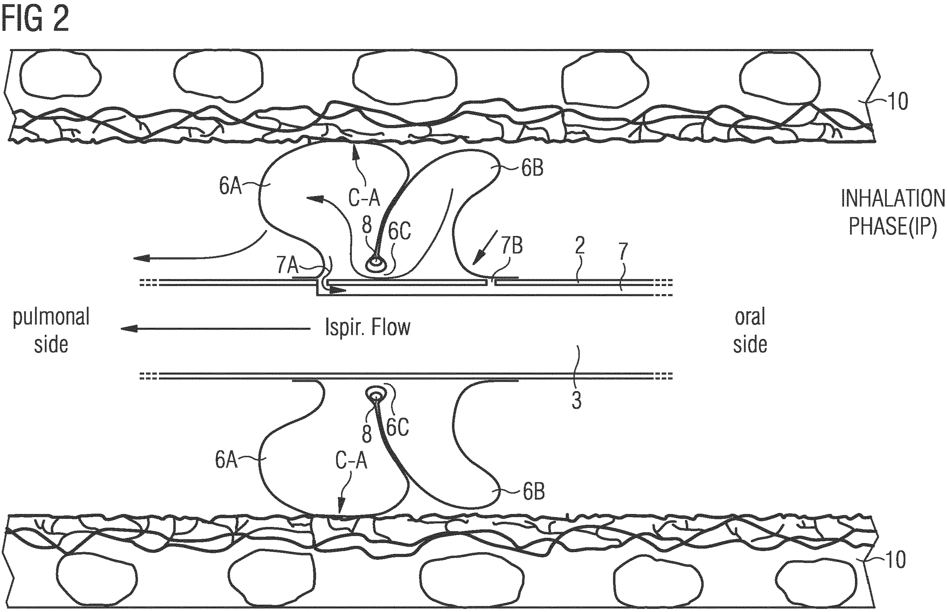

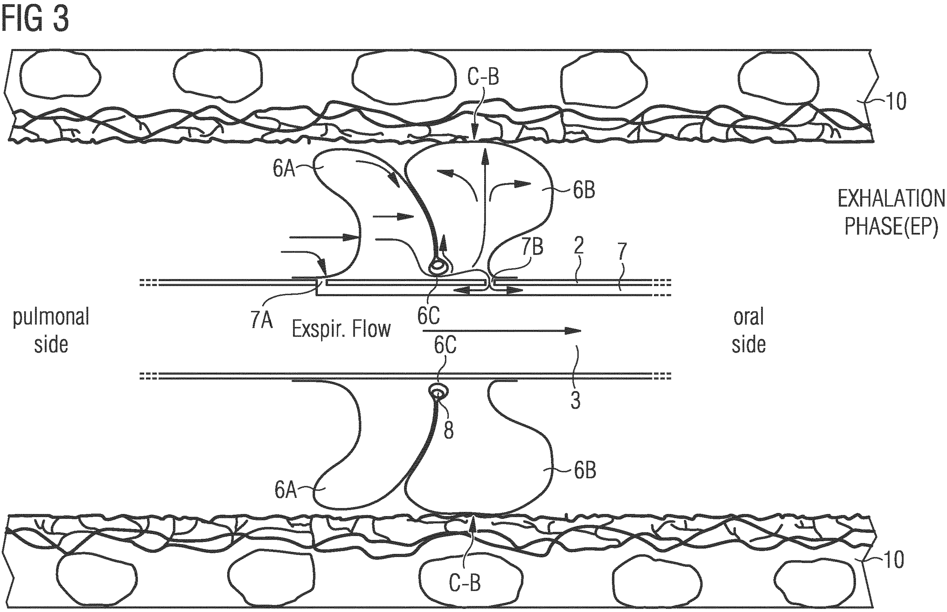

Fig. 1 shows a possible exemplary embodiment of a tracheal tube 1 according to a first aspect of the present invention. The tracheal tube 1 comprises a tube shaft 2 having a central lumen 3 to provide the ventilation of a patient in respiratory cycles of the ventilated patient by a ventilator of a breathing apparatus connected at an proximate end to an oral side of the tube shaft 2 inserted in the traches of the patient. In the illustrated embodiment of Fig. 1, the tube shaft 2 is connected at its oral side to the breathing apparatus by means of a connector 4 which can be fixed by fixing means 5 to a proximate end of the tube shaft 2. The tracheal tube 1 as illustrated in Fig. 1 comprises a sealing cuff 6 surrounding the tube shaft 2. The sealing cuff 6 is inflatable with a fluid to seal off the patient's trachea in an inflated state of the sealing cuff 6 at a compression area where the sealing cuff 6 is pressed against the mucosa 10 of the patient's trachea as also illustrated in Figs2, 3. The sealing cuff 6 is divided in the illustrated embodiment of Fig. 1 in two torus-shaped sealing cuff compartments 6A, 6B which are adapted to exchange a fluid F with each other through a fluid passage 6C during the ventilation of the patient such that a compression area C is moved along the mucosa 10 of the patient's trachea during the respiratory cycle of the patient. The shape and the length of the tube shaft 2 can vary depending on the use case. In the illustrated embodiment of Fig. 1, the tube shaft 2 comprises a slightly bent shape facilitating the introduction of the tube shaft 2 into the trachea of the patient.

The tracheal tube 1 comprises a fluid inflation channel 7 which can be used to inflate the sealing cuff compartments 6A, 6B of the sealing cuff 6 with a fluid F after insertion of the tracheal tube 1 into the patient's trachea before starting ventilation of the patient. In a preferred embodiment, the fluid inflation channel 7 comprises for each sealing cuff compartment 6A, 6B of the sealing cuff 6 an associated internal fluid channel opening 7A, 7B as illustrated in Fig. 1. The internal fluid channel openings 7A, 7B can form a passage between the fluid inflation channel 7 and the respective sealing cuff compartment 6A, 6B and is adapted to support the exchange of the fluid F between the sealing cuff compartments 6A, 6B of the sealing cuff 6 during ventilation of the patient.

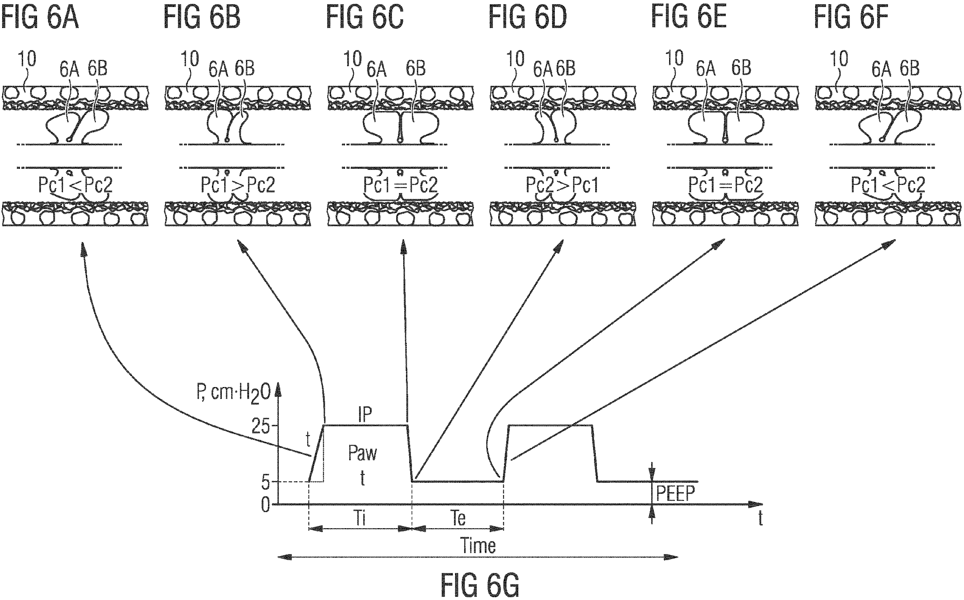

The tracheal tube 1 further comprises at least one constriction element 8 configured to divide the sealing cuff 6 into two torus-shaped sealing cuff compartments 6A, 6B. The constriction element 8 can for instance comprise an elastic ring adapted to divide the sealing cuff 6 into two torus-shaped sealing cuff compartments 6A, 6B as illustrated in Fig. 1. In the illustrated embodiment of Fig. 1, the sealing cuff 6 is divided in two sealing cuff compartments 6A, 6B. However, the number of sealing cuff compartments 6A, 6B can vary depending on the use case. In an alternative embodiment, the sealing cuff 6 may comprise three or more sealing cuff compartments each having an associated internal fluid channel opening to the fluid inflation channel 7. The compression area is provided over time by alternating sealing cuff compartments 6A, 6B of the sealing cuff during ventilation of the patient as also illustrated in Fig. 6. After insertion of the tracheal tube 1 into the trachea of the patient, the sealing cuff 6 and its sealing cuff compartments 6A, 6B can be inflated by a fluid F through the fluid inflation channel 7. The fluid F used for inflating the sealing cuff 6 can comprise a liquid or a gas or a gas mixture. The gas mixture can comprise in particular also narcotic gases.

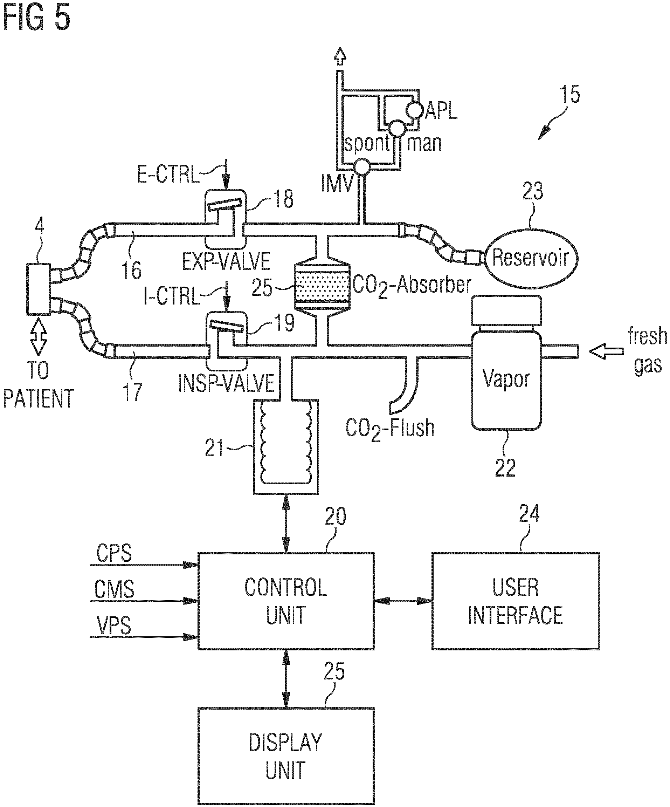

In a possible embodiment, a physical pressure within the sealing cuff compartments 6A, 6B of the sealing cuff 6 can be controlled by a control unit of a breathing apparatus. This can be achieved by controlling an inhalation valve 19 and/or an exhalation valve 18 connected to the sealing cuff compartments 6A, 6B of the sealing cuff 6 through the fluid inflation channel 7 of the tracheal tube 1 as also shown in Fig.5. The physical pressure within the inflated sealing cuff 6 can be changed dynamically over time during ventilation of the patient by a ventilator 21 integrated in the breathing apparatus 15. The ventilation of the patient is performed through the central lumen 3 of the tube shaft 2.

In a possible embodiment, an inflation actuator 9 attached to the fluid inflation channel 7 as illustrated in Fig. 1 can be used to inflate the sealing cuff 6 of the tracheal tube 1 after insertion of the tracheal tube 1 into the patient's trachea through the fluid inflation channel 7. The inflation actuator 9 can in a possible embodiment be manually operated by a user. After insertion of the tracheal tube 1, a fluid F such as a liquid or gas is pressed into the sealing cuff 6 through the fluid inflation channel 7. When inserting the tracheal tube 1 into the patient's trachea, the sealing cuff 6 is in a not yet in an inflated state. By pressing the fluid F by means of the inflation actuator 9 into the sealing cuff 6 through the fluid inflation channel 7, the volume of the sealing cuff compartments 6A, 6B is filled with fluid F and expands. Accordingly, by using the inflation actuator 9, it is possible to reach the inflated state of the sealing cuff 6. In a possible embodiment, the inflation actuator 9 can be connected to the sealing cuff 6 by means of an elastic tube as illustrated in Fig. 1. In a possible implementation, the elastic tube can after insertion of the tracheal tube 1 be disconnected from the inflation actuator 9 and manually connected to an interface of the breathing apparatus. An integrated pressure sensor of the breathing apparatus can be used to generate a cuff pressure signal CPS(t) as a first monitoring signal representing a pressure change of a physical pressure P within the sealing cuff 6. The pressure sensor can convert the physical pressure at the proximate end of the elastic fluid inflation channel 7 into an electrical cuff pressure signal CPS (t) which represents a pressure change dP/dt of a physical pressure P within the sealing cuff 6 connected through the inflation channel 7 to the pressure sensor over time. The generated cuff pressure signal CPS (t) can be supplied to a control unit of the breathing apparatus during ventilation of the patient by a ventilator of the breathing apparatus through the central lumen 3 of the tube shaft 2 of the tracheal tube 1.

In a further possible embodiment, the tracheal tube 1 comprises itself an integrated pressure sensor element configured to generate a cuff pressure signal CPS(t) representing a pressure change of a physical pressure within the sealing cuff 6 during ventilation of the patient. The cuff pressure signal CPS generated by the integrated pressure sensor element can also be supplied to the control unit of the breathing apparatus connected to the tracheal tube 1 by means of the connector element 4. In this embodiment, the tracheal tube 1 comprises a first signal interface to transmit the generated cuff pressure signal CPS generated by the integrated pressure sensor element of the tracheal tube 1 to the control unit of the breathing apparatus.

In a further possible embodiment of the tracheal tube 1 according to the first aspect of the present invention, the constriction element 8 can be adapted to induce an electrical signal in a wire loop integrated in the tube shaft 2 or attached to the tube shaft 2 in response to a lateral oscillating movement of the constriction element 8 along the tube shaft 2 during ventilation of the patient. In this embodiment, the tracheal tube 1 is also capable of generating a cuff movement signal CMS (t) representing the lateral oscillating movement of the constriction element 8 along the tube shaft 2. This induced cuff movement signal CMS can be supplied by the tracheal tube 1 as a second monitoring signal through a second signal interface of the tracheal tube 1 to the control unit of the breathing apparatus. Accordingly, in a possible embodiment, the control unit of the breathing apparatus receives a cuff pressure signal CPS through a first signal interface of the tracheal tube 1 and a cuff movement signal CMS through a second signal interface of the tracheal tube 1. Both signals CPS, CMS can be displayed on a display unit of the breathing apparatus to a user such as a physician. Further, the cuff pressure signal CPS and the cuff movement signal CMS can be used to support monitoring and controlling of the ventilation of the patient. In a possible implementation, the constriction ring 8 can comprises an electrical conductive material to generate an induced electrical signal applied through the second signal interface of the tracheal tube 1 to the control unit of the breathing apparatus. Further a ventilation pressure signal VPS reflecting the inner pressure of the ventilation air in the central lumen of the tube shaft 2 can be supplied via a third signal interface of the tracheal tube 1 to the control unit of the breathing apparatus.

The tracheal tube 1 can comprise in a possible embodiment towards a pulmonary side a first sealing cuff compartment 6A and towards an oral side a second sealing cuff compartment 6B as shown in Fig. 1. During an inhalation phase IP of the respiratory cycle, the patient's trachea is sealed off by the first sealing cuff compartment 6A of the sealing cuff 6. During an exhalation phase EP of the respiratory cycle, the patient's trachea is sealed off by the second sealing cuff compartment 6B of the sealing cuff 6.

In a preferred embodiment, the sealing cuff 6 comprising the sealing cuff compartments 6A, 6B is rotationally symmetric around a length axis of the tube shaft 2 of the tracheal tube 1. In a possible embodiment, the sealing cuff 6 is replaceable in an uninflated state of the sealing cuff 6. In this embodiment, the sealing cuff 6 can be exchanged easily by another sealing cuff 6 in its uninflated state. Further, in a possible implementation, a maximum volume of the sealing cuff 6 in its inflated state can also be adjustable by a provided clamping mechanism. The sealing cuff 6 can comprise a thin wall made of a flexible elastic material such as latex, silicone, polyvinyl chloride and/or polyurethane. In a possible embodiment, the sealing cuff 6 can comprise a membrane made of an elastic material being impermeable against narcotic gases. In a further possible embodiment, the membrane can be made of a transparent material. The fluid inflation channel 7 can be either integrated in the tube shaft 2 or can be attached to the tube shaft 2. The tube inflation channel 7 can comprise a tube or a pipe made of a flexible material as also illustrated in the embodiment of Fig. 1.

The tracheal tube 1 according to the present invention can comprise an endotracheal tube or a tracheostomy tube. Tracheostomy is a hole that surgeons can make through the front of the neck and into the windpipe (trachea) of the patient. The tracheostomy tube can be placed into the hole to keep it open for breathing of the patient. The sealing cuff 6 at the distal end of the tracheostomy tube 1 can be inflated as illustrated in Fig. 1 and reduces the chance of material being inhaled or aspirated into the lungs of the patients by offering airway protection. In contrast to tracheostomy tubes, endotracheal tubes are inserted through the mouth or nose of the ventilated patient. The tracheal tube 1 with a replaceable sealing cuff 6 allows to use different kinds of sealing cuffs for different use cases, in particular sealing cuffs 6 with different internal diameters adapted to the diameter of the respective trachea of the patient.

Figs. 2, 3 illustrate the operation of a tracheal tube 1 according to the first aspect of the present invention.

Fig. 2 illustrates the operation of the tracheal tube 1 during an inhalation phase IP where air is pressed by the breathing apparatus through the central lumen 3 of the tracheal tube 1 into the patient's lung. As can be seen in Fig. 2, during the inhalation phase IP, the first sealing cuff compartment 6A at the pulmonary side of the tracheal tube 1 is sealing off the patient's trachea at a compression area C-A. The pressure P-A within the first sealing cuff compartment 6A is higher than the physical pressure P-B within the second sealing cuff compartment 6B facing the oral side of the tracheal tube 1. During the inhalation phase IP as shown in Fig. 2, the first sealing cuff compartment 6A is fully inflated and seals off the patient's trachea. As shown in Fig. 2, the inflated first sealing cuff compartment 6A presses against the mucosa 10 of the patient's trachea at a compression area C-A. The second sealing cuff compartment 6B facing the oral side does not press against the mucosa 10 of the patient's trachea during the inhalation phase IP as illustrated in Fig. 2. During the inhalation phase IP, fluid F is transferred from the second sealing cuff compartment 6B through the fluid passage 6C beneath the constriction element 8 to the first sealing cuff compartment 6A on the pulmonary side.

Fig. 3 illustrates a situation during an exhalation phase EP of the respiratory cycle. During the exhalation phase EP, the direction of the flow of the ventilated breathing air through the central lumen 3 of the tube shaft 2 is reversed. A lung of the patient constricts and presses the air through the central lumen 3 back to the breathing apparatus. The fluid F is migrating from the first sealing cuff compartment 6A on the pulmonary side through the fluid passage 6C to the second sealing cuff compartment 6B on the oral side as illustrated in Fig. 3. The second sealing cuff compartment 6B is inflated because of the increased pressure and presses against the mucosa 10 at a compression area C-B.

As can be seen from Figs. 2, 3, during the respiratory cycle RCYCLE of the patient, the compression area C is moved along the mucosa 10 of the patient's trachea. Accordingly, the sealing cuff 6 does not press against the mucosa 10 of the patient's trachea at a fixed location but the compression area C is moving along the mucosa 10 of the patient's trachea. This has the advantage that the blood circulation of the trachea 10 is stimulated and a damaging of the tissue of the mucosa 10 of the trachea is avoided even if the inner pressure P within the inflated sealing cuff compartment of the sealing cuff 6 exceeds the predefined maximum cuff pressure threshold of 20 to 30 cm H2O. Accordingly, even if the physical pressure P within the sealing cuff 6 exceeds a predetermined mucosal perfusion threshold, damaging of the trachea's tissue can be avoided because of the moveable compression area C. Moving of the compression area C in length direction of the tube shaft 2 is achieved by the exchange of fluid F between the different sealing cuff compartments 6A, 6B of the sealing cuff 6 through the fluid passage 6C as illustrated in Figs. 2, 3. This exchange of fluid F can in a possible embodiment be supported by the provision of the fluid channel openings 7A, 7B forming a passage between the fluid inflation channel 7 and the respective sealing cuff compartment 6A, 6B. Each sealing cuff compartment 6A, 6B of the sealing cuff 6 can comprise in a possible embodiment an associated internal fluid channel opening 7A, 7B forming a passage between the fluid inflation channel 7 and the respective sealing cuff compartment 6A, 6B being adapted to support the exchange of the fluid F between the sealing cuff compartments 6A, 6B of the sealing cuff 6 during ventilation of the patient. As becomes evident from Figs. 2, 3, the compression area C is provided over time by alternating sealing cuff compartments 6A, 6B of the sealing cuff 6 during the ongoing ventilation of the patient. The physical pressure P within the inflated sealing cuff 6 can be changed dynamically over time during ventilation of the patient. Each sealing cuff compartment 6A, 6B of the sealing cuff 6 comprises an associated internal physical pressure. This physical pressure of the sealing cuff compartment does change dynamically over time during the respiratory cycle RCYCLE of the ventilated patient. The compression area C performs during the respiratory cycles an oscillating movement along the inner surface of the mucosa 10 of the patient's trachea. This lateral oscillating movement of the compression area C can be captured by observing the lateral oscillating movement of the constriction element 8. In a possible embodiment, the constriction element 8 can comprise a constriction ring including an electrical conductive material. The lateral oscillating movement of the constriction element 8 can be captured by generation of an induced electrical signal representing the lateral oscillating movement of the conductive constriction element 8 during the respiratory cycle RCYCLE. This induced electrical signal can be supplied via an electrical wire loop integrated in the tube shaft 2 of the tracheal tube 1 to a second signal interface of the tracheal tube 1 and can be supplied as a cuff movement signal CMS to a control unit of the breathing apparatus. Besides the cuff movement signal CMS, the control unit of the breathing apparatus can also receive a cuff pressure signal CPS through another signal interface of the tracheal tube 1. This cuff pressure signal CPS can in a possible embodiment be generated by a pressure sensor element configured to generate a cuff pressure signal CPS representing a pressure change of the physical pressure within the sealing cuff 6 during ventilation of the patient over time. In a possible implementation, an integrated pressure sensor element can be provided for each sealing cuff compartment 6A, 6B of the sealing cuff 6. In an alternative embodiment, a single integrated pressure sensor element is provided for the sealing cuff 6.

In a still further alternative embodiment, a pressure sensor can be integrated in the breathing apparatus and detect the physical pressure at the proximate end of the fluid inflation channel 7 of the tracheal tube 1.

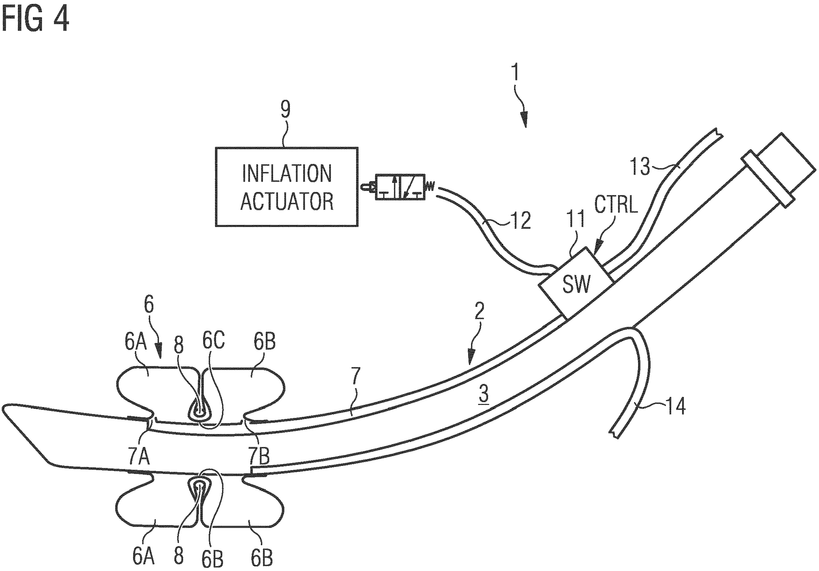

In the illustrated embodiment of Fig. 4, the tracheal tube 1 comprises a controllable valve 11 which can be provided at its proximate end. The controllable valve 11 can be controlled by a control unit of the breathing apparatus. The controllable valve 11 of the tracheal tube 1 can be used to switch the fluid inflation channel 7 between the inflation actuator 9 and a pressure sensor integrated in the breathing apparatus. In a first switching state of the controllable valve 11, the fluid inflation channel 7 is connected through a channel 12 to the inflation actuator 9. The inflation actuator 9 is adapted to inflate the sealing cuff 6 of the tracheal tube 1 after insertion of the tracheal tube 1 into the patient's trachea by pressing a fluid F via the fluid inflation channel 7 into the sealing cuff compartments 6A, 6B of the sealing cuff 6. After the sealing cuff 6 has been inflated, the controllable valve 11 can be switched into another switching state where the fluid inflation channel 7 is connected through a tube 13 to a pressure sensor integrated in the breathing apparatus. The pressure sensor integrated in the breathing apparatus is adapted to generate a cuff pressure signal CPS representing the pressure change of the physical pressure within the sealing cuff 6. The generated cuff pressure signal CPS is supplied by the pressure sensor of the control unit of the breathing apparatus during ventilation of the patient. The control unit of the breathing apparatus can control a switching of the controllable switch valve 11 during the operation of the breathing apparatus between two operation modes, i.e. an inflation mode and a pressure measuring mode..

In the illustrated embodiment of Fig. 4, the tracheal tube 1 further comprises a suction channel 14. The suction channel 14 can be used to extract unwanted secretions from the area of the sealing cuff 6.

Fig. 5 illustrates schematically a possible exemplary embodiment of a breathing apparatus 15 according to the second aspect of the invention. The breathing apparatus 15 can be connected to the tracheal tube 1 through a connector element 4 as also illustrated in Fig. 1. The central lumen 3 of the tracheal tube 1 is connected through the connector element 4 to an expiration path 16 and to an inspiration path 17 within the breathing apparatus 15. The expiration path 16 can comprise a controllable expiration valve 18 as shown in Fig. 5. Further, the inspiration path 17 can comprise a controllable inspiration valve 19 as also illustrated in Fig. 5. The expiration valve 18 and the inspiration valve 19 may be controlled in a possible embodiment by a control unit 20 of the breathing apparatus 15. The control unit 20 is connected to a ventilator 21 used for ventilation of a patient through the tracheal tube 1. Fresh ventilation gas can be vaporized by a vaporization unit 22 before being pressed through the inspiration valve 19 of the inspiration path 17 into the central lumen 3 of the connected tracheal tube 1. The expired air can be supplied by the central lumen 3 of the tracheal tube 1 to the expiration path 16 and can be either be supplied to a reservoir 23 or output to the environment. In a possible embodiment, the breathing apparatus 15 may also include a CO2 absorber 24 as shown in Fig. 5.

In a possible embodiment, the expiration valve 18 and the inspiration valve 19 may form a return valve without requiring control signals from the control unit 20. In this embodiment, the ventilation is performed by the controlled ventilator 21.

In the illustrated embodiment of Fig. 5, the breathing apparatus 15 further comprises a user interface 24 and a display unit 25. The user interface 24 is provided for receiving user commands by a user such as a surgeon or physician. The control unit 20 connected to the user interface 24 is adapted to control the ventilator 21. Further, the control unit 20 can in a possible embodiment also be used to control the expiration valve 18 and/or the inspiration valve 19 of the breathing apparatus 15. The display unit 25 of the breathing apparatus 15 is adapted in a possible embodiment to display a cuff pressure signal CPS(t) representing a pressure change dP/dt of the physical pressure P within the sealing cuff 6 of the tracheal tube 1 connected to the breathing apparatus 15 during ventilation of the patient. In a possible embodiment, the control unit 20 receives the cuff pressure signal CPS through a first signal interface from the connected tracheal tube 1. In a still further possible embodiment, the control unit 20 may also receive a cuff movement signal CMS through a second signal interface of the tracheal tube 1. In a possible embodiment, the display unit 25 can also be adapted to display the cuff movement signal CMS to a user such as a surgeon during ventilation of the patient.

The displayed cuff pressure signal CPS assists the user during ventilation of the patient, in particular to adjust the physical pressure within the sealing cuff compartments 6A, 6B of the sealing cuff 6 precisely. The cuff movement signal CMS which can also be displayed on the display unit 25 of the breathing apparatus 15 allows the user to monitor the oscillating movement of the compression area C along the inner surface of the patient's trachea. In this way, a user can also observe a drift of the oscillating cuff movement signal CMS indicating an unwanted displacement of the tracheal tube 1 with respect to the patient's trachea. The cuff pressure signal CPS indicating the physical pressure within the sealing cuff 6 or sealing cuff compartment 6A, 6B can comprise a periodic signal such as sine wave signal synchronized with the respiratory cycle of the breathing apparatus 15. Also, the cuff movement signal CMS can comprise a periodic signal, in particular a sine wave signal synchronized with the respiratory cycle of the breathing apparatus 15.

The cuff pressure signal CPS and the cuff measurement signal CMS received by the control unit 20 of the breathing apparatus 15 through the signal interfaces of the tracheal tube 1 can be processed by a signal processing unit of the control unit 20 to derive further relevant information during ventilation of the patient. The frequency of the cuff pressure signal CPS and of the cuff movement signal CMS can also be displayed on the display unit 25 of the breathing apparatus 15 to a user. Further, the processing unit can determine a drift of the CPS signal and the CMS signal over time and may trigger a warning signal displayed on the display unit 25, for instance if the drift of the CMS signal indicates an unwanted relative movement between the tracheal tube 1 and the mucosa 10. Such a drift may indicate that the pressure P within the inflated sealing cuff 6 is too low and/or that the sealing cuff 6 does slip within the trachea of the patient. In this scenario, the control unit 20 can trigger an increase of the internal pressure within the sealing cuff 6 by pressing additional fluid F through the stitch 11 an the fluid inflation channel 7 into the sealing cuff compartments 6A, 6B of the sealing cuff 6 as a countermeasure. Further, the sealing cuff pressure signal CPS indicating the internal physical pressure within the sealing cuff 6 can indicate if a membrane of a sealing cuff compartment 6A, 6B of the sealing cuff 6 is leaking. In this scenario, the amplitude of the sealing cuff pressure signal CPS is significantly reduced indicating a leaking sealing cuff 6. In this case, it may be required to replace the sealing cuff 6 to guarantee a tight sealing of the trachea during ventilation of the patient.

In a possible embodiment, the sealing cuff 6 placed on the tube shaft 2 of the tracheal tube 1 may be tested in a testing phase before insertion into the trachea of the patient. The sealing cuff 6 can be inflated through the fluid insertion channel 7 by means of the inflation actuator 9. Then, the cuff pressure signal CPS can be observed to determine whether the internal physical pressure P within the sealing cuff compartments 6A, 6B is stable or not. If the pressure amplitude of the cuff pressure signal CPS is diminishing rapidly, this indicates that the sealing cuff 6 is leaking. In this case, the faulty sealing cuff 6 is replaced by another sealing cuff 6 and the tracheal tube 1 is inserted into the trachea of the patient after successful testing of the replaced sealing cuff 6. Monitoring of the cuff pressure signal CPS and the cuff movement signal CMS provides additional safety during ventilation of a patient. The pressure of the ventilation air provided by the ventilator 21 of the breathing apparatus 15 and supplied to the lung of the patient through the central lumen 3 of the tracheal tube 1 can be regulated in a possible embodiment depending on the monitored cuff pressure signal CPS and/or depending on the monitored cuff movement signal CMS. By evaluating the cuff pressure signal CPS, it is possible to fine-tune the physical pressure within the sealing cuff compartments 6A, 6B of the sealing cuff, in particular to avoid a too high compression pressure at the compression area C between the surface of the inflated sealing cuff compartment 6A, 6B and the internal surface of the trachea of the patient.

Fig. 6 illustrates different phases of the ventilation of a patient during a respiratory cycle. During an inhalation phase IP as illustrated in Fig. 6A and as also illustrated in Fig. 2, ventilation air is pressed through the central lumen 3 of the tracheal tube 1 towards the lung of the patient wherein the sealing cuff compartment 6A on the pulmonary side is inflated. Accordingly, the pressure within the first sealing cuff compartment 6A is increased and exceeds the pressure of the second oral side sealing cuff compartment 6B. In this process, the compression area C moves towards the pulmonary side of the tracheal tube 1 along the mucosa 10 of the patient's trachea. Fig. 6C illustrates a neutral position where both sealing cuff compartments 6A, 6B comprise the same inner physical pressure. During the exhalation phase EP as also illustrated in Fig. 3, the compression area C moves along the mucosa 10 of the patient's trachea towards the oral side and the pressure within the second oral side sealing cuff compartment 6B increases until the second sealing cuff compartment 6B is inflated and seals off the trachea at the compression area C as illustrated in Fig. 6D. From the expiration phase EP, the sealing cuff is further deformed back to the shape illustrated in Fig. 6F in a neutral state (illustrated in Fig. 6E) to start a new respiratory cycle in an inhalation phase IP.

Fig. 6G illustrates the corresponding waveform of the pressure signal VPS(t) of the ventilation air VA within the central lumen 3 of the tracheal tube 1 over time. As illustrated in the example of Fig. 6D, the pressure within the central lumen 3 during the inspiration phase IP is 25 cm H2O and is reduced during the expiration phase EP through a positive end expiratory pressure PEEP of about 5 cm H2O. Because of the moveable compression area, the physical pressure may even exceed the mucosal perfusion threshold pressure of 30 to 40 cm H2O during the inhalation phase IP or during the exhalation phase EP because this pressure is not applied permanently on the same spot or point of the mucosa 10 of the patient's trachea.

In a possible embodiment, the pressure within the central lumen 3 of the connected tracheal tube 1 can also be measured by an associated pressure sensor provided within the central lumen 3 of the tracheal tube 1 and supplied as a third monitoring signal via a third signal interface of the tracheal tube 1 to the control unit 20 of the breathing apparatus 15. The signal waveform illustrated in Fig. 6G can also be displayed on the display unit 25 along with the cuff pressure signal CPS and the cuff movement signal CMS. A signal processing unit of the control unit 20 can be adapted to process the cuff pressure signal CPS, the cuff movement signal CMS and the ventilation pressure signal VPS illustrated in Fig. 6G in real time to determine automatically any deviations from the normal signal behavior during ventilation and to fine-tune the local pressure within the sealing cuff compartments 6A, 6B of the sealing cuff 6 during the ventilation. The processing can comprise a Fast Fourier Transformation FFT of the periodic monitoring signals CPS, CMS, VPS to calculate their respective spectrum in the frequency domain. In a possible embodiment the spectrum of the signals CPS, CMS, VPS is displayed on the display unit 25 of the breathing apparatus 15. In a further implementation of the processing unit can determine further relevant signal parameters including a phase shift between the signals CPS, CMS,VPS, average values, time periods and a momentary frequency. An abnormal signal behavior of a signal can trigger a warning signal displayed to a user and countermeasures. The signals CPS, CMS, VPS can be logged in a data memory of the breathing apparatus 15 for further processing and evaluation. The signals CPS, CMS, VPS can also be stored locally along with other patient data of the ventilated patient in a data memory connected to a control and data interface of the tracheal tube 1. If the ventilated patient enters a critical health state it can immediately determined by the medical personal whether the ventilation and/or the tracheal tube 1 operate correctly on the basis of the displayed signals CPS,CMS, VPS.

The first signal interface for the cuff pressure signal CPS, the second interface for the cuff movement signal CMS, and the third signal interface for the air ventilation pressure signal VPS can form part of a data and control interface between the tracheal tube 1 and the breathing apparatus 15. The data and control interface can be implemented by a plug and a socket.

In a preferred embodiment a separate portable display unit can be connected to data and control interface of the tracheal tube 1 and fixed to the tracheal tube 1.This is helpful if the breathing apparatus 15 does not have a fitting interface for receiving the signals, CPS, CMS, VPS.

The data and control interface of the tracheal tube 1 used for transmitting the monitoring signals CPS,CMS,VPS can comprise a wired or a wireless interface.

While the invention may be susceptible to various modifications and alternative forms, specific embodiments have been shown by way of example in the drawings and have been described in detail herein. However, it should be understood that the invention is not intended to be limited to the particular forms disclosed. Rather, the invention is to cover all modifications, equivalents, and alternatives falling within the scope of the present invention as defined by the claims. Indeed, the present technique may not only be applied to form cuffs for tracheal tubes but for any type of devices designed for insertion into a human or animal body to which a tight seal is desired.

## Claims

1. A tracheal tube (1) for ventilation of a patient, said tracheal tube (1) comprising:
a tube shaft (2) having a central lumen (3) to provide the ventilation of the patient in respiratory cycles of the patient by a ventilator of a breathing apparatus (15) connected to the tube shaft(2);
a sealing cuff (6) surrounding the tube shaft (2) and being inflatable with a fluid to seal off the patient's trachea in an inflated state of the sealing cuff (6) at a compression area where the sealing cuff (6) is pressed against the mucosa (10) of the patient's trachea, wherein the sealing cuff (6) is divided into torus-shaped sealing cuff compartments (6A,6B) which are adapted to exchange fluid with each other through a fluid passage (6C) during the ventilation of the patient such that the compression area is moved along the mucosa (10) of the patient's trachea during a respiratory cycle of the patient.

2. The tracheal tube according to claim 1 comprising a fluid inflation channel (7) used to inflate the sealing cuff compartments of the sealing cuff (6)with the fluid after insertion of the tracheal tube into the patient's trachea before ventilation of the patient, wherein the fluid inflation channel (6) has for each sealing cuff compartment (6A,6B) of the sealing cuff (6) an associated internal fluid channel opening (7A,7B) forming a passage between the fluid channel (7) and the respective sealing cuff compartment(6A,6B) and adapted to support the exchange of the fluid between the sealing cuff compartments (6A,6B) of the sealing cuff (6) during ventilation of the patient.

3. The tracheal tube according to claim 1 and 2 comprising at least one constriction element (8) configured to divide the sealing cuff (6) into the torus-shaped sealing cuff compartments (6A, 6B).

4. The tracheal tube according to any of the preceding claims 1 to 3 wherein the compression area is provided over time by alternating sealing cuff compartments (6A,6B) of the sealing cuff (6) during ventilation of the patient.

5. The tracheal tube according to any of the preceding claims 1 to 4 wherein the fluid comprises a liquid or gas, in particular a narcotic gas.

6. The tracheal tube according to any of the preceding claims 1 to 5 wherein a physical pressure within the sealing cuff compartments (6A,6B) of the sealing cuff is controlled by a control unit (20) of a breathing apparatus (15) by controlling a ventilator (25),an inhalation valve (19) and/or an exhalation valve (18) connected to the sealing cuff compartments (6A,6B) of the sealing cuff (6) through the fluid inflation channel (7) of the tracheal tube (1).

7. The tracheal tube according to any of the preceding claims 1 to 6 wherein a physical pressure within the inflated sealing cuff (6) is changed dynamically over time during ventilation of the patient by the ventilator (21) of the breathing apparatus (15) through the central lumen (2) of the tube shaft (3) of the tracheal tube(1)

8. The tracheal tube according to any of the preceding claims 1 to7 wherein the fluid inflation channel (7) comprises at its proximate end a controllable valve (11) to switch the fluid inflation channel between an inflation actuator (9) adapted to inflate the sealing cuff (6) of the tracheal tube (1) after insertion of the tracheal tube into the patient's trachea through the fluid inflation channel (7) and a pressure sensor of the breathing apparatus (1) adapted to supply a cuff pressure signal representing a pressure change of a physical pressure within the sealing cuff (6) to a control unit (20) of the breathing apparatus (1) during ventilation of the patient by the ventilator (21) of the breathing apparatus (1) through the tube shaft (2) of the tracheal tube (1).

9. The tracheal tube according to any of the preceding claims 1 to 8 comprising an integrated pressure sensor element configured to generate a cuff pressure signal (CPS) representing a pressure change of a physical pressure within the sealing cuff (6) during ventilation of the patient.

10. The tracheal tube according to claim 9 comprising a first signal interface to supply the cuff pressure signal (CPS) generated by the integrated pressure sensor element of the tracheal tube (1) to a control unit (20) of the breathing apparatus (1) connected to the tracheal tube (1) .

11. The tracheal tube according to any of the preceding claims 3 to 10 wherein the constriction element (8) is adapted to induce an electrical signal in a wire loop integrated in the tube shaft (2) or attached to the tube shaft (2) in response to a lateral oscillating movement of the constriction element (8) along the tube shaft (2) during ventilation of the patient.

12. The tracheal tube according to claim 11 comprising a second signal interface connected to the wire loop and adapted to supply the induced electrical signal representing the lateral oscillating movement of the constriction element along the tube shaft (2) as a cuff movement signal (CMS) to a control unit (20) of the breathing apparatus (1) connected to the tracheal tube (1).

13. The tracheal tube according to any of the preceding claims 3 to 12 wherein the constriction element (8) comprises a constriction ring including electrical conductive material.

14. The tracheal tube according to any of the preceding claims 1 to 13 comprising at a pulmonary side a first sealing cuff compartment (6A) and at an oral side a second sealing cuff compartment (6B) , wherein during an inhalation phase (IP) of the respiratory cycle the patient's trachea is sealed off by the first sealing cuff compartment (6A) of the sealing cuff and wherein during an exhalation phase (EP) of the respiratory cycle the patient's trachea is sealed off by the second sealing cuff compartment (6B) of the sealing cuff (6).

15. The tracheal tube according to any of the preceding claims 1 to 14 comprising a endotracheal tube or a tracheostomy tube.

16. The tracheal tube according to any of the preceding claims 1 to 15 wherein the sealing cuff (6) is replaceable in an uninflated state of the sealing cuff (6).

17. The tracheal tube according to any of the preceding claims 1 to 16 wherein a maximum volume of the sealing cuff (6) in its inflated state is adjustable by a clamping mechanism.

18. The tracheal tube according to any of the preceding claims 1 to 17 comprising a third signal interface connected to a pressure sensor integrated in the central lumen (3) of the tube shaft (2) and adapted to supply a ventilation pressure signal (VPS) to a control unit (20) of the breathing apparatus (1) connected to the tracheal tube (1).

19. The tracheal tube according to any of the preceding claims wherein the first signal interface for the cuff pressure signal (CPS) and/or the second signal interface for the cuff movement signal (CMS) and/or the third signal interface for the ventilation pressure signal (VPS) form part of a data- and control interface of the tracheal tube(1).

20. The tracheal tube according to claim 19 comprising a display unit connected to the data and control interface of the tracheal tube(1) and adapted to display the cuff pressure signal (CPS), the cuff movement signal (CMS) and/or the ventilation pressure signal (VPS).

21. A breathing apparatus (15) comprising
a ventilator (21) used for ventilation of a patient through a tracheal tube (1) according to any of the preceding claims 1 to 20, said breathing apparatus (1) comprising a control unit (20)configured to control the ventilator (21), an inhalation valve (19) and/or an exhalation valve (18) of the breathing apparatus (1) and comprising a display unit (25) adapted to display a cuff pressure (CPS) signal representing a pressure change of a physical pressure within the sealing cuff (6) of the tracheal tube (1) connected to the breathing apparatus (1) during ventilation of the patient and/or adapted to display a cuff movement signal (CMS) representing the lateral movement of the sealing cuff(6) along the trachea during ventilation of the patient and/or adapted to display a ventilation air pressure signal (VPS) representing a pressure of the ventilation air measured in a central lumen (3) of the tracheal tube(1)
